# FASCICULE DE BREVET EUROPEEN

(11) **EP 2 981 213 B1**
(45) Date de publication et mention de la délivrance du brevet: **13.12.2017**
(21) Numéro de dépôt: 14719647.1
(22) Date de dépôt: 31.03.2014
(51) Int. Cl.: A61B 6/00, G06T 7/00, G06T 7/40

(54) **PROCÉDÉ ET SYSTÈME DE CARACTÉRISATION D'UN TISSU OSSEUX**
VERFAHREN UND SYSTEM ZUR KENNZEICHNUNG EINES KNOCHENGEWEBES
METHOD AND SYSTEM FOR CHARACTERISING A BONE TISSUE

(30) Priorité: 03.04.2013 FR 1353003
(43) Date de publication de la demande: 10.02.2016
(73) Titulaire: Medimaps Group SA, 1228 Plan-les-Ouates, Geneve (CH)
(72) Inventeur: CHAINTREUIL, Jean, décédé (FR)
(74) Mandataire: Pontet Allano & Associes
(86) Numéro de dépôt international: PCT/EP2014/056486
(87) Numéro de publication internationale: WO 2014/161827

(56) Documents cités:
- EP-A1- 1 283 492
- FR-A1- 2 960 762
- US-A1- 2004 114 726
- US-A1- 2006 062 442
- US-A1- 2007 047 794

## Description

### Domaine technique

La présente invention concerne un procédé de caractérisation d'un tissu osseux. Elle concerne également un système mettant en oeuvre un tel procédé.

Le domaine de l'invention est le domaine médical et plus précisément le domaine de l'étude des tissus osseux avec des appareils d'imagerie comprenant un émetteur de rayons de particules électriquement neutres et mettant en oeuvre un capteur de rayons. L'invention s'applique plus particulièrement au domaine de l'imagerie médicale de haute précision et de l'étude de la texture des os.

### Etat de la technique

Actuellement, il existe de nombreux appareils d'imagerie de tissus osseux. Ces appareils comprennent une source de particules électriquement neutres prévue pour émettre un rayonnement et éventuellement un récepteur doté d'un capteur. Le tissu osseux est positionné entre l'émetteur et le récepteur.

Le procédé mis en oeuvre dans ces appareils comprend une émission d'un rayonnement de particules électriquement neutres qui viennent frapper le tissu osseux. Les particules électriquement neutres traversent plus ou moins le milieu étudié en fonction de sa densité. L'atténuation est plus importante dans un milieu osseux que dans les tissus mous.

L'image obtenue par ces procédés est une image en niveaux de gris et le niveau de gris en un point de l'image obtenue dépend de la quantité de particules frappant le capteur en ce point. L'image en niveaux de gris est ensuite analysée et traitée pour déterminer la valeur d'un ou plusieurs paramètres relatifs au milieu osseux, aussi appelé « paramètre de caractérisation » dans la présente demande. Un tel paramètre de caractérisation peut par exemple être le paramètre de texture H, aussi connu sous le nom de paramètre de Hurst.

Une caractérisation correcte des tissus osseux, et notamment des os, suppose de diminuer, voire annuler, les effets des tissus mous entourant l'os sur l'image réalisée. En effet, une difficulté majeure de la caractérisation des os est d'obtenir un résultat qui est indépendant de la quantité et du type de tissus mous entourant l'os à caractériser et qui introduisent des interférences dans l'image et donc dans la détermination d'un paramètre de caractérisation.

Par ailleurs, la Demanderesse a constaté que les particules électriquement neutres qui atteignent le capteur en un point donné comprenant d'une part des particules incidentes provenant directement de l'émetteur et ayant traversé le tissu osseux et les tissus mous en regard de ce point donné et d'autre part des particules électriquement neutres diffusées par d'autres parties du tissu osseux et des tissus mous qui ne se trouvent pas en regard du point donné. Ces particules électriquement neutres diffusées introduisent également des interférences qui se retrouvent sur l'image en niveaux de gris et donc sur la valeur d'un paramètre de caractérisation.

Les procédés et systèmes actuels de caractérisation de tissus osseux réalisent une caractérisation du tissu osseux sans prendre en compte ces interférences et fournissent des résultats approximatifs qui dans certains cas peuvent être aberrants.

Le document FR 2 960 762 A1 décrit un procédé de caractérisation d'un tissu osseux comprenant une acquisition d'une image avec un paramètre d'émission déterminé en fonction d'une valeur de niveaux de gris déterminée dans une première image. Le document US 2004/114726 A1 décrit le calibrage d'un indice de densité minérale osseuse par une mesure de niveaux de gris obtenus pour différentes épaisseurs d'un fantôme. Le document US 2007/047794 A1 décrit un procédé d'analyse d'images radiologiques pouvant comporter une étape de calibrage à l'aide d'un fantôme. Le document US 2006/062442 A1 décrit un procédé d'analyse d'un paramètre de structure osseuse afin de prédire un risque de fracture.

Un but de l'invention est de pallier les inconvénients cités ci-dessus.

Un autre but de l'invention est de proposer un procédé et système de caractérisation d'un tissu osseux permettant de diminuer voire de s'affranchir des interférences causées par les tissus mous et/ou par le rayonnement diffusé lors de la caractérisation du tissu osseux.

Un autre but de la présente invention est de proposer un procédé et système de caractérisation d'un tissu osseux permettant de déterminer la valeur d'un paramètre de caractérisation indépendamment de l'appareil utilisé, en particulier du capteur utilisé, et/ou des conditions de caractérisation.

Enfin, un autre but de l'invention est de proposer un procédé et système de caractérisation d'un tissu osseux réalisant une caractérisation plus précise que les procédés et systèmes actuels.

### Exposé de l'invention

L'invention propose d'atteindre au moins l'un de ces buts par un procédé de caractérisation d'un tissu osseux avec un appareil d'imagerie comprenant un émetteur de rayons et mettant en oeuvre un capteur de rayons, ledit procédé étant caractérisé en ce qu'il comprend les étapes suivantes :
- acquisition d'au moins une image en niveaux de gris dudit tissu osseux avec ledit appareil d'imagerie à une valeur (MAS₁), dite de mesure, d'un paramètre d'émission relatif aux rayons émis,
- mesure, par analyse de ladite image :
   - d'une valeur (SD₁), dite mesurée, d'une déviation standard des niveaux de gris,
   - d'une valeur (GLA₁), dite mesurée, d'un niveau de gris moyen, et
   - d'une valeur, dite mesurée, d'un paramètre de caractérisation (H₁) dudit tissu osseux ;
- calibration, dite cible, de ladite valeur mesurée (H₁) du paramètre de caractérisation par rapport à une valeur (GLA_{cible}), dite cible, d'un niveau de gris moyen prédéterminé pour ledit capteur, ladite calibration fournissant une valeur (H_{cible}), dite cible, dudit paramètre de caractérisation,
- calibration, dite fixe, de ladite valeur mesurée (SD₁) de la déviation standard des niveaux de gris par rapport à une valeur (MAS_{fixe}), dite fixe, du paramètre d'émission prédéterminée, ladite calibration fournissant une valeur, dite fixe (SD_{fixe}) de la déviation standard des niveaux de gris, et
- détermination d'une valeur (H_{corrigé}), dite corrigée, de la valeur dudit paramètre de caractérisation en fonction de ladite valeur cible (H_{cible}) dudit paramètre de caractérisation, et de ladite valeur

fixe (SD_{fixe}) et d'une valeur cible (SD_{cible}) de la déviation standard des niveaux de gris.

Le procédé selon l'invention propose donc de réaliser une caractérisation d'un tissu d'abord par mesure de la valeur d'un paramètre de caractérisation puis par correction de la valeur mesurée.

La correction de la valeur mesurée comprend une étape de calibration cible permettant de référencer la valeur mesurée par rapport à une valeur cible du niveau de gris moyen pour le capteur utilisé, et ainsi de corriger la mesure réalisée par rapport au capteur. La valeur cible (GLA_{cible}) du niveau de gris moyen, permet de ramener la mesure réalisée dans une plage de fonctionnement recommandé par le constructeur. En effet, une image trop « sombre » traduit une trop faible puissance d'émission et une image trop « claire » traduit une trop forte puissance d'émission, aucune de ces images ne présentant un contraste suffisant pour déterminer correctement le paramètre de caractérisation.

La correction comprend en outre une étape de calibration fixe permettant de référencer la mesure réalisée par rapport à une valeur fixe préalablement choisi du paramètre d'émission relatif à la quantité de particules émise, permettant de corriger la mesure réalisée par rapport aux conditions de mesure.

La Demanderesse a découvert que la quantité de photons diffusés dans une image en niveaux de gris est fonction de valeurs de la dispersion des niveaux de gris dans cette image. La demanderesse a également découvert de manière surprenante que les interférences introduites par les photons diffusés dans la détermination d'un paramètre de caractérisation peuvent être corrigées en prenant en compte d'une part deux valeurs relatives aux niveaux de gris pour deux valeurs d'un même paramètre d'émission de l'émetteur et d'autre part une constante qui peut être préalablement déterminée de manière empirique.

La mise en oeuvre du procédé nécessite l'acquisition d'une unique image à une valeur de mesure d'un paramètre d'émission de l'émetteur et la mesure d'une valeur relative aux niveaux de gris.

Ainsi, le procédé selon l'invention permet de réaliser une caractérisation d'un tissu osseux, par exemple de la texture de l'os, en limitant voire en s'affranchissant des interférences causées par le rayonnement diffusé, par le type de capteur utilisé et par les conditions de prise de l'image en niveaux de gris.

La quantité et la nature du tissu mou entourant l'os étant une des causes, voir la cause principale, du rayonnement diffusé le procédé selon l'invention permet également de s'affranchir des interférences du tissu mou dans la caractérisation d'un tissu osseux.

Avantageusement l'image en niveau de gris peut être réalisée par un détecteur numérique. L'image en niveau de gris peut alternativement être réalisée de manière analogique ou sur film radiologique. Dans ce dernier cas, le procédé selon l'invention comprend en outre une étape de numérisation d'une telle image analogique, réalisée sur film radiologique par exemple.

Avantageusement, la valeur cible (GLA_{cible}) du niveau de gris moyen est choisie en fonction du capteur, et plus particulièrement de la plage des niveaux de gris pouvant être obtenu par le capteur utilisé.

Plus particulièrement, la valeur cible (GLA_{cible}) du niveau de gris moyen choisi pour un capteur donné est autour de, en particulier égale à, la valeur médiane de niveau de gris pour le capteur donné.

Par exemple, pour un capteur de marque Hamamatsu® proposant 4096 niveaux de gris, la valeur GLA_{cible} choisie peut être de 2000.

Avantageusement, la valeur fixe (MAS_{fixe}) du paramètre d'émission peut être choisie proche de la limite inférieure des valeurs de paramètre d'émission observées en imagerie clinique pour un modèle de capteur. Par exemple, la valeur fixe (MAS_{fixe}) du paramètre d'émission choisie peut être de 10mAs, alors que la valeur de mesure (MAS₁) utilisée pour l'acquisition de l'image peut être 15mAs.

Avantageusement, l'étape de calibration cible peut en outre comprendre une détermination de la valeur cible (SD_{cible}) de la déviation standard des niveaux de gris en fonction de la valeur de mesure (MAS₁) du paramètre d'émission et d'une valeur cible (MAS_{cible}) prédéterminée dudit paramètre d'émission.

La valeur cible (SD_{cible}) de la déviation standard des niveaux de gris peut avantageusement être déterminée selon la relation suivante : $S {D}_{cible} = S {D}_{1} \left(\frac{MA {S}_{cible}}{MA {S}_{1}}\right)$ avec :
- SD_{cible} la valeur cible de la déviation standard des niveaux de gris,
- SD₁ la valeur mesurée de la déviation standard des niveaux de gris,
- MAS_{cible} une valeur cible du paramètre d'émission, et
- MAS₁ la valeur de mesure du paramètre d'émission.

Préalablement à la détermination de la valeur cible (SD_{cible}) de la déviation standard des niveaux de gris, l'étape de de calibration peut comprendre une détermination de la valeur cible (MAS_{cible}) du paramètre d'émission en fonction de la valeur de mesure du paramètre d'émission (MAS₁), de la valeur mesurée (GLA₁) du niveau de gris moyen et de la valeur cible (GLA_{cible}) prédéterminée du niveau de gris moyen pour le capteur utilisé.

La valeur cible (MAS_{cible}) du paramètre d'émission peut avantageusement être déterminée selon la relation suivante : $MA {S}_{cible} = MA {S}_{1} \left(\frac{GL {A}_{cible} - cte}{GL {A}_{1} - cte}\right)$ avec :
- MAS_{cible} la valeur cible du paramètre d'émission,
- MAS₁ la valeur mesurée du paramètre d'émission,
- GLA_{cible} la valeur cible du niveau de gris moyen,
- GLA₁ la valeur mesurée du niveau de gris moyen, et
- cte une constante dont la valeur est proche du nombre maximum de niveaux de gris disponibles pour un modèle de détecteur, plus ou moins un ajustement spécifique du détecteur.

La constante « cte » peut être préalablement déterminée avec exactitude, de manière empirique, par une série de mesures.

Avantageusement, la valeur fixe (SD_{fixe}) de la déviation standard des niveaux de gris peut être déterminée selon la relation suivante : $S {D}_{fixe} = S {D}_{1} \left(\frac{MA {S}_{fixe}}{MA {S}_{1}}\right)$ avec :
- SD_{fixe} la valeur fixe de la déviation standard des niveaux de gris,
- SD₁ la valeur mesurée de la déviation standard des niveaux de gris,
- MAS_{fixe} la valeur fixe du paramètre d'émission, et
- MAS₁ la valeur de mesure du paramètre d'émission.

L'image en niveaux de gris est acquise autour d'une région, dite d'intérêt, préalablement identifiée.

L'identification de la région d'intérêt peut être réalisée par prise d'une image préalable, en particulier en niveaux de gris, et par identification, sur cette image préalable, d'au moins un marqueur anatomique.

Selon un mode de réalisation préféré de l'invention, le paramètre de caractérisation du tissu osseux peut être le paramètre H, appelé également paramètre de Hurst.

Dans un exemple de réalisation particulier, la valeur de H peut être dérivée du mouvement brownien fractionnaire défini dans le domaine fréquentiel par l'intégrale stochastique suivante : ${B}_{H} \left(t\right) = \frac{1}{2 π} {\int }_{- ∞}^{+ ∞} \frac{1}{{\left(i ω\right)}^{H + 1 / 2}} \left({e}^{it ω} - 1\right) dB \left(ω\right)$ pour 0 < H < 1, B(ω) étant le mouvement brownien complexe. H ne pouvant pas être déduit de cette intégrale, il est déterminé par la méthode de l'estimation par maximum de vraisemblance (EMV).

L'estimation de H par maximum de vraisemblance peut être réalisée à partir du vecteur des incréments (bruit gaussien fractionnaire (bgf)) du signal, G₁, (G₁(t) = B_{H}(t+1) -B_{H}(t)). En effet, le bgf est stationnaire, gaussien centré et sa densité de probabilité paramétrée par H et C s'écrit : $P \left({G}_{1} ; H , C\right) = \frac{1}{{\left(2 π\right)}^{\left(\frac{N}{2}\right)} {| R |}^{\frac{1}{2}}} exp \left(- \frac{1}{2} {G}_{1}^{T} {R}^{- 1} {G}_{1}\right) ,$ où R est la matrice d'autocorrélation de G₁ qui dépend de H et de la constante C.

L'estimation par maximum de vraisemblance correspond à la valeur de H pour laquelle cette probabilité est maximale. Le logarithme de la fonction de vraisemblance permet de simplifier cette expression : $Log \left(P \left({G}_{1} ; H , C\right)\right) = - \frac{N}{2} Log \left(2 π\right) - \frac{1}{2} Log \left(| R |\right) - \frac{1}{2} {G}_{1}^{T} {R}^{- 1} {G}_{1} .$

R peut être décomposée en C R'. Dans ces conditions l'équation ci-dessus devient : $Log \left(P \left({G}_{1} ; H , C\right)\right) = - \frac{N}{2} Log \left(2 π\right) - \frac{N}{2} Log \left(C\right) - \frac{1}{2} Log \left(| R ' |\right) - \frac{1}{2 C} {G}_{1}^{T} R {'}^{- 1} {G}_{1} .$

Le maximum de cette expression par rapport à C peut être trouvé en annulant la dérivée partielle correspondante. On obtient alors : $\frac{\partial Log \left(P \left({G}_{1} ; H , C\right)\right)}{\partial C} = 0 _ C = \frac{{G}_{1}^{T} R {'}^{- 1} {G}_{1}}{N}$

En substituant la valeur de C ainsi obtenue dans l'équation et en négligeant les termes constants, nous obtenons la fonction à maximiser : $Log \left(P \left({G}_{1} ; H , C\right)\right) = - N Ln \left(\frac{{G}_{1}^{T} R {'}^{- 1} {G}_{1}}{N}\right) - Ln _ R ' _ .$

La forme de l'estimateur n'étant pas explicite, le maximum de la probabilité doit être calculé par une méthode numérique. Cette recherche se fait par la méthode du nombre d'or qui consiste à chercher le maximum dans un intervalle borné (dans notre cas [0, 1]) et permet de trouver le maximum au bout d'un nombre d'itérations fixé pour une précision donnée. A chaque itération l'intervalle de recherche est réduit par un facteur égal au nombre d'or. Le calcul du déterminant et de l'inverse de la matrice d'autocorrélation se fait à l'aide de l'algorithme de Levinson.

Dans le cas où le paramètre de caractérisation du tissu osseux, est H, la valeur cible (H_{cible}) du paramètre de caractérisation peut avantageusement être déterminée selon la relation suivante : ${H}_{cible} = {H}_{1} + ( \left(GL {A}_{cible} - GL {A}_{1}\right) . \left(\left(a {.10}^{- b}\right) - \left(c {.10}^{- d .} \left(GL {A}_{cible} + GL {A}_{1}\right)\right)\right)$ avec :
- H_{cible} la valeur cible du paramètre H,
- H₁ la valeur mesurée du paramètre H,
- GLA_{cible} la valeur cible du niveau de gris moyen,
- GLA₁ la valeur mesurée du niveau de gris moyen, et
- « a », « b », « c » et « d » des valeurs constantes, spécifiques du modèle de détecteur, déterminées par régression quadratique de H en fonction de GLA sur des fantômes de calibration.

De plus, la valeur corrigée (H_{corrigé}) du paramètre H peut avantageusement être déterminée selon la relation suivante : ${H}_{corrigée} = {H}_{cible} + e \left(\frac{1}{S {D}_{fixe}} - \frac{1}{S {D}_{cible}}\right)$ avec :
- H_{corrigée} la valeur corrigée du paramètre H,
- H_{cible} la valeur cible du paramètre H,
- SD_{cible} la valeur cible de la variation standard du niveau de gris,
- SD_{fixe} la valeur fixe de la déviation standard des niveaux de gris, et
- « e» une valeur constante.

Dans chacune des relations données dans la présente demande, au moins une valeur constante est une valeur préalablement déterminée, de manière empirique, pour un capteur donné et pour un paramètre de caractérisation donné, éventuellement pour une tension d'alimentation donnée de l'émetteur et/ou un tissu à caractériser, ou encore une zone anatomique à caractériser.

Chaque valeur constante, peut être déterminée en réalisant une pluralité de mesures avec des sujets ou des objets pour lesquels la valeur du paramètre de caractérisation est connue.

Par exemple, la valeur de la constante « e »peut être déterminée lors d'une phase comprenant les étapes suivantes :
- mesures du paramètre de caractérisation, par exemple du paramètre H, pour des objets en plastique d'épaisseur 0, 0.5cm, 1cm, 2cm, 3cm et 4cm, ou de certains d'entre eux.
- pour chaque mesure i, détermination de la pente « eᵢ » de la courbe reliant H_{corrigée, i} à la quantité $\left(\frac{1}{S {D}_{fixe}} - \frac{1}{S {D}_{cible}}\right) ,$ et
- moyenne des valeurs de « eᵢ ».

Pour un capteur de marque Hamamatsu® proposant 4096 niveaux de gris et pour l'imagerie du calcanéum, e=9.6.

Selon un autre exemple particulier, le paramètre de caractérisation peut être la densité de tissus osseux.

Selon un autre aspect de l'invention il est proposé un système comprenant des moyens agencés et programmés pour mettre en oeuvre les étapes du procédé selon l'invention.

Un tel système comprend :
- un appareil d'imagerie médicale comprenant un émetteur et mettant en oeuvre un récepteur,
- un module de commande,
- des moyens de traitement pour traiter des images en niveaux de gris pour déterminer les différentes mesurées décrites plus haut, et
- des moyens de calculs agencés pour déterminer les différentes valeurs corrigées, fixes et cibles décrites plus haut.

Le module de commande peut être programmé pour communiquer avec et commander les moyens de traitement, les moyens de calculs et l'émetteur.

Le module de commande, le module de traitement et les moyens de calculs peuvent être intégrés, au moins partiellement, dans l'appareil d'imagerie médical.

Dans sa définition générale, la présente invention exploite la relation pour un objet donné entre la proportion de rayonnement diffusé dans la dose reçue par le détecteur et l'écart-type des niveaux de gris sur une image en niveaux de gris.

Par conséquent, tout paramètre de caractérisation d'un objet dont la mesure est sensible au rayonnement diffusé pourrait être déterminé/corrigé selon la présente invention.

### Description des figures et modes de réalisation

D'autres avantages et caractéristiques apparaîtront à l'examen de la description détaillée d'un mode de réalisation nullement limitatif, et des dessins annexés sur lesquels
- la figure 1 est une représentation sous la forme d'un diagramme des étapes d'un exemple de procédé de caractérisation selon l'invention ;
- la figure 2 est une représentation schématique d'un système selon l'invention ; et
- la figure 3 est une représentation d'un exemple de résultats obtenus avec le procédé et le système selon l'invention.

Il est bien entendu que les modes de réalisation qui seront décrits dans la suite ne sont nullement limitatifs. On pourra notamment imaginer des variantes de l'invention ne comprenant qu'une sélection de caractéristiques décrites par la suite isolées des autres caractéristiques décrites, si cette sélection de caractéristiques est suffisante pour conférer un avantage technique ou pour différencier l'invention par rapport à de l'état de la technique antérieur. Cette sélection comprend au moins une caractéristique de préférence fonctionnelle sans détails structurels, ou avec seulement une partie des détails structurels si c'est cette partie qui est uniquement suffisante pour conférer un avantage technique ou pour différencier l'invention par rapport à l'état de la technique antérieur.

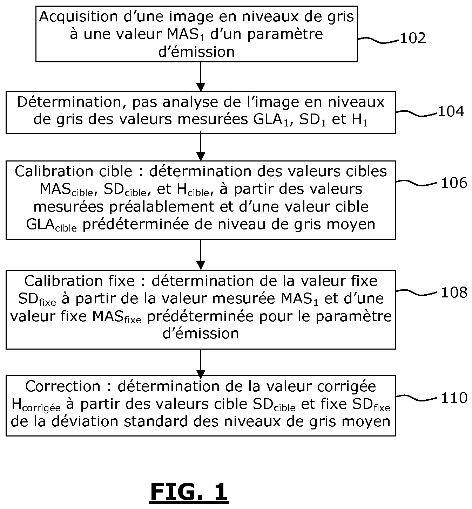

La figure 1 est une représentation sous la forme d'un diagramme des étapes d'un exemple de procédé d'imagerie selon l'invention pour l'imagerie du calcanéum mettant en oeuvre un capteur de rayons de marque Hamamatsu® proposant 4096 niveaux de gris.

Dans l'exemple présent, le paramètre de caractérisation dont la valeur est recherchée est le paramètre de Hurst H. Le paramètre d'émission est la dose de photons délivrée par l'émetteur en mAs. Ce paramètre sera désigné par MAS dans la suite.

Le procédé 100 représenté sur la figure 1 comprend une première étape 102 d'acquisition d'une image en niveaux de gris avec une valeur de MAS₁ = 15mAs.

A l'étape 104, le procédé 100 réalise la détermination d'une valeur, dite mesurée, pour chacun des paramètres suivants par analyse de l'image en niveaux de gris :
- GLA₁ : valeur mesurée du niveau de gris moyen sur l'image en niveaux de gris ;
- SD₁ : valeur mesurée de la déviation standard des niveaux de gris sur l'image en niveaux de gris ; et
- H₁ déterminé tel que décrit précédemment, c'est-à-dire comme la dérivée du mouvement brownien fractionnaire déterminée par la méthode de l'estimation par maximum de vraisemblance.

Le procédé 100 comprend en outre une première étape 106 de calibration, dite calibration cible, réalisant une calibration de la mesure réalisée par rapport à une valeur cible, GLA_{cible}, du niveau de gris moyen. Cette valeur cible GLA_{cible} de niveau de gris moyen est préalablement choisie pour le capteur utilisé pour l'acquisition de l'image en niveaux de gris à l'étape 102. Dans le cadre de cet exemple, le capteur utilisé est un capteur de rayons de marque Hamamatsu® proposant 4096 niveaux de gris. La valeur cible GLA_{cible} choisi est 2000.

L'étape 106 de calibration cible permet de déterminer les valeurs cibles avec les relations suivantes : $S {D}_{cible} = S {D}_{1} \left(\frac{MA {S}_{cible}}{MA {S}_{1}}\right)$ $MA {S}_{cible} = MA {S}_{1} \left(\frac{GL {A}_{cible} - cte}{GL {A}_{1} - cte}\right)$ ${H}_{cible} = {H}_{1} + ( \left(GL {A}_{cible} - GL {A}_{1}\right) . \left(\left(a {.10}^{- b}\right) - \left(c {.10}^{- d} . \left(GL {A}_{cible} + GL {A}_{1}\right)\right)\right)$ avec « a », « b », « c », « d » et « cte » des valeurs constantes préalablement déterminées de manière empirique pour un capteur donné, un tissu osseux à caractériser. Ces valeurs, spécifiques du modèle de détecteur, peuvent être déterminées par régression quadratique de H en fonction de GLA sur des fantômes de calibration dont les valeurs de H et de GLA sont mesurées pour différentes doses de rayonnement (mAs).

Cette étape de calibration cible, réalisée par rapport au niveau de gris moyen, permet de ramener la mesure réalisée, lors de l'étape 102, à une plage de niveaux de gris exploitable et conseillé pour le capteur utilisé, et permet ainsi de rendre la mesure indépendante des plages de mesure utilisées par un même capteur. Par conséquent, la calibration cible permet d'éviter des erreurs de mesure pouvant être causées par une mesure qui serait décalée dans un sens ou dans l'autre par rapport à une plage de mesure optimisée pour le capteur.

Le procédé 100 comprend en outre une première étape 108 de calibration, dite calibration fixe, réalisant une calibration de la mesure réalisée par rapport à une valeur fixe, MAS_{fixe}, du paramètre d'émission, c'est-à-dire de la quantité de particules émises pour réaliser l'acquisition de l'image. Cette valeur fixe MAS_{fixe} du paramètre d'émission est préalablement choisie proche de la limite inférieure des valeurs de paramètre d'émission observées en imagerie clinique pour un modèle de capteur donné. Dans le cadre de cet exemple, le capteur utilisé est un capteur de rayons de marque Hamamatsu® proposant 4096 niveaux de gris. La valeur fixe MAS_{fixe} choisie est 10mAs.

Lors de cette étape de calibration la valeur fixe SD_{fixe} de la déviation standard des niveaux de gris est déterminée. Cette valeur SD_{fixe} sera ensuite utilisée pour corriger la valeur mesurée H₁ du paramètre de Hurst. La valeur fixe SD_{fixe} est déterminée selon la relation suivante : $S {D}_{fixe} = S {D}_{1} \left(\frac{MA {S}_{fixe}}{MA {S}_{1}}\right)$

Cette étape de calibration fixe, réalisée par rapport au paramètre d'émission, permet de ramener la mesure réalisée lors de l'étape 102 à une condition d'éclairage fixe du tissu osseux à imager, et ainsi de rendre la mesure réalisée indépendante des conditions d'éclairage utilisées pour la mesure.

Le procédé 100 représenté sur le FIGURE 1 comprend ensuite une étape 110 de correction de la valeur mesurée H₁ du paramètre de caractérisation, qui est dans l'exemple présent le paramètre de Hurst.

L'étape de correction 110 permet de déterminer la valeur corrigée H_{corrigée} avec la relation suivante : ${H}_{corrigée} = {H}_{cible} + e \left(\frac{1}{S {D}_{fixe}} - \frac{1}{S {D}_{cible}}\right)$ avec « e » une constante.

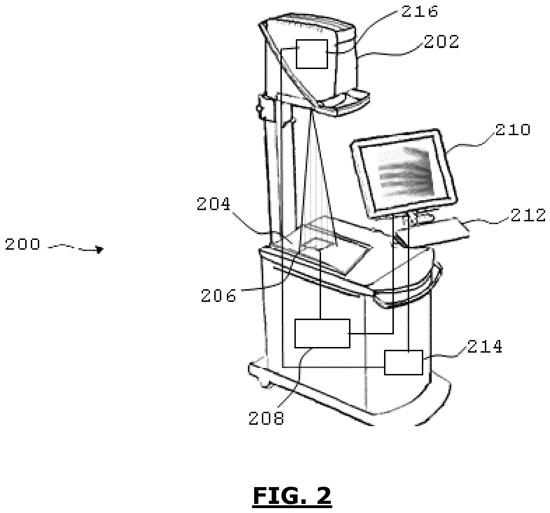

La figure 2 est une représentation schématique d'un exemple de système d'imagerie 200 selon l'invention.

Le système d'imagerie 200 est mobile. Il comprend un émetteur 202 de rayons de particules chargées et un récepteur 204 muni d'un capteur 206.

Le capteur 206 est relié à un module 208 de génération d'image en niveaux de gris. Les images générées par le module de génération d'image 208 sont affichées sur un écran d'affichage 210.

Le système 200 comprend en outre des moyens 212 de sélection d'une région d'intérêt sur l'image affichée sur l'écran d'affichage 210.

Le système 200 comprend par ailleurs un module 214 de calcul pour calculer la valeur de niveaux de gris moyen dans les images réalisées ainsi que la valeur de la déviation standard des niveaux de gris, la valeur d'au moins un paramètre de caractérisation et la valeur d'un paramètre d'émission en fonction de relations préalablement renseignées.

La valeur du paramètre d'émission est fournie à un module 216 de commande qui permet de modifier et d'ajuster la valeur de ce paramètre au niveau de l'émetteur 202.

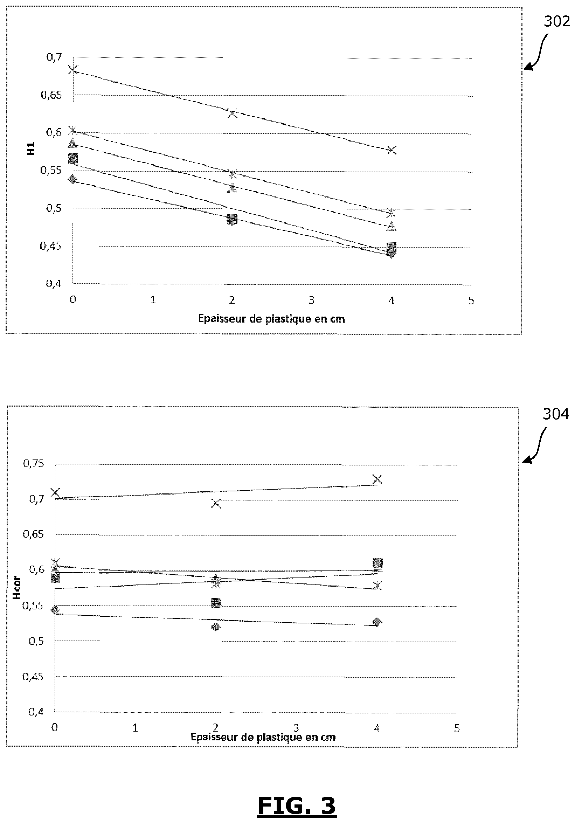

La figure 3 est une représentation schématique d'un exemple non limitatif de résultats obtenus avec le procédé et le système selon l'invention.

Les courbes 302 montrent la valeur H₁, du paramètre H mesurée pour des doses d'émission différentes et pour une épaisseur variable d'une couche de plastique disposée entre l'émetteur et le tissu osseux à caractériser. Cette couche de plastique introduit du rayonnement diffusé parasite, capté par le détecteur et présent dans l'image en niveau de gris, et dont la quantité dépend directement de l'épaisseur de la couche de plastique.

Les courbes 304 montrent pour chaque configuration la valeur H_{cor}, du paramètre H corrigée selon l'invention.

Sur les courbes données, l'axe horizontal représente l'épaisseur de la couche de plastique en cm et l'axe vertical la valeur du paramètre H, à savoir, la valeur mesurée H₁ pour les courbes 302 et la valeur corrigée H_{cor} pour les courbes 304.

Les courbes 302 montrent clairement que la valeur mesurée H₁ mesurées pour un même tissu osseux n'est pas constante et diminue lorsque l'épaisseur de la couche plastique, disposée entre le tissu osseux et l'émetteur, augmente. Cette variation de la valeur mesurée H₁ est causée par le rayonnement diffusé généré par la couche de plastique et la dose utilisée. En comparaison, sur les courbes 304, la valeur corrigée H_{cor} du paramètre H est sensiblement constante, et ne dépend plus de l'épaisseur de la couche plastique utilisée, ni de la dose utilisée en émission.

L'invention n'est bien sûr pas limitée aux exemples qui viennent d'être décrits.

## Revendications

1. Procédé (100) de caractérisation d'un tissu osseux avec un appareil (300) d'imagerie comprenant un émetteur de rayons (202) et mettant en oeuvre un capteur de rayons (204), ledit procédé comprenant les étapes suivantes :
- acquisition (102) d'au moins une image en niveaux de gris dudit tissu osseux avec ledit appareil d'imagerie à une valeur (MAS₁), dite de mesure, d'un paramètre d'émission relatif aux rayons émis,
- mesure (104), par analyse de ladite image :
• d'une valeur (GLA₁), dite mesurée, d'un niveau de gris moyen, et
• d'une valeur, dite mesurée, d'un paramètre de caractérisation (H₁) dudit tissu osseux ;
le procédé étant **caractérisé en ce qu'**il comprend en outre les étapes suivantes :
- mesure (104), par analyse de ladite image, d'une valeur (SD₁), dite mesurée, d'une déviation standard des niveaux de gris,
- calibration, dite cible, de ladite valeur mesurée (H₁) du paramètre de caractérisation par rapport à une valeur (GLA_{cible}), dite cible, d'un niveau de gris moyen prédéterminée pour ledit capteur, ladite calibration fournissant une valeur (H_{cible}), dite cible, dudit paramètre de caractérisation,
- calibration, dite fixe, de ladite valeur mesurée (SD₁) de la déviation standard des niveaux de gris par rapport à une valeur (MAS_{fixe}), dite fixe, du paramètre d'émission prédéterminée, ladite calibration fournissant une valeur, dite fixe (SD_{fixe}) de la déviation standard des niveaux de gris, et
- détermination d'une valeur (H_{corrigé}), dite corrigée, de la valeur dudit paramètre de caractérisation en fonction de ladite valeur cible (H_{cible}) dudit paramètre de caractérisation, et de ladite valeur fixe (SD_{fixe}) et d'une valeur cible (SD_{cible}) de la déviation standard des niveaux de gris.

2. Procédé (100) selon la revendication 1, **caractérisé en ce qu'**il comprend en outre l'étape de calibration cible comprend en outre une détermination de la valeur cible de la déviation standard des niveaux de gris en fonction de la valeur de mesure (MAS₁) du paramètre d'émission et d'une valeur cible (MAS_{cible}) prédéterminée dudit paramètre d'émission.

3. Procédé (100) selon la revendication 2, **caractérisé en ce que** l'étape de calibration cible comprend en outre une détermination de la valeur cible (MAS_{cible}) du paramètre d'émission en fonction de la valeur de mesure du paramètre d'émission (MAS₁), de la valeur mesurée (GLA₁) du niveau de gris moyen et de la valeur cible (GLA_{cible}) prédéterminée du niveau de gris moyen pour le capteur utilisé.

4. Procédé (100) selon l'une quelconque des revendications précédentes, **caractérisé en ce que** l'image est acquise autour d'une région, dite d'intérêt, préalablement identifiée sur l'image, par au moins un marqueur anatomique.

5. Procédé (100) selon l'une quelconque des revendications précédentes, **caractérisé en ce que** la valeur fixe (SD_{fixe}) de la déviation standard des niveaux de gris est déterminée selon la relation suivante : $S {D}_{fixe} = S {D}_{1} \left(\frac{MA {S}_{fixe}}{MA {S}_{1}}\right)$ avec :
- SD_{fixe} la valeur fixe de la déviation standard des niveaux de gris,
- SD₁ la valeur mesurée de la déviation standard des niveaux de gris,
- MAS_{fixe} la valeur fixe du paramètre d'émission, et
- MAS₁ la valeur de mesure du paramètre d'émission.

6. Procédé (100) selon l'une quelconque des revendications 2 à 5, **caractérisé en ce que** la valeur cible (SD_{cible}) de la déviation standard des niveaux de gris est déterminée selon la relation suivante : $S {D}_{cible} = S {D}_{1} \left(\frac{MA {S}_{cible}}{MA {S}_{1}}\right)$ avec :
- SD_{cible} la valeur cible de la déviation standard des niveaux de gris,
- SD₁ la valeur mesurée de la déviation standard des niveaux de gris,
- MAS_{cible} la valeur cible du paramètre d'émission, et
- MAS₁ la valeur de mesure du paramètre d'émission.

7. Procédé (100) selon l'une quelconque des revendications 3 à 6, **caractérisé en ce que** la valeur cible (MAS_{cible}) du paramètre d'émission est déterminée selon la relation suivante : $MA {S}_{cible} = MA {S}_{1} \left(\frac{GL {A}_{cible} - cte}{GL {A}_{1} - cte}\right)$ avec :
- MAS_{cible} la valeur cible du paramètre d'émission,
- MAS₁ la valeur mesurée du paramètre d'émission,
- GLA_{cible} la valeur cible du niveau de gris moyen,
- GLA₁ la valeur mesurée du niveau de gris moyen, et
- cte une constante.

8. Procédé (100) l'une quelconque des revendications précédentes, **caractérisé en ce que** le paramètre de caractérisation est le paramètre H, appelé également paramètre de Hurst.

9. Procédé (100) selon la revendication précédente, **caractérisé en ce que** la valeur cible (H_{cible}) du paramètre de caractérisation est déterminée selon la relation suivante : ${H}_{cible} = {H}_{1} + ( \left(GL {A}_{cible} - GL {A}_{1}\right) . \left(\left(a {.10}^{- b}\right) - \left(c {.10}^{- d} . \left(GL {A}_{cible} + GL {A}_{1}\right)\right)\right)$ avec :
- H_{cible} la valeur cible du paramètre H,
- H₁ la valeur mesurée du paramètre H,
- GLA_{cible} la valeur cible du niveau de gris moyen,
- GLA₁ la valeur mesurée du niveau de gris moyen, et
- a, b, c et d des valeurs constantes.

10. Procédé (100) selon l'une quelconque des revendications 8 ou 9, **caractérisé en ce que** la valeur corrigée (H_{corrigé}) du paramètre de caractérisation est déterminée selon la relation suivante : ${H}_{corrigée} = {H}_{cible} + e \left(\frac{1}{S {D}_{fixe}} - \frac{1}{S {D}_{cible}}\right)$ avec :
- H_{corrigée} la valeur corrigée du paramètre H,
- H_{cible} la valeur cible du paramètre H,
- SD_{cible} la valeur cible de la variation standard du niveau de gris,
- SD_{fixe} la valeur fixe de la déviation standard des niveaux de gris, et
- e une valeur constante.

11. Procédé (100) selon l'une quelconque des revendications 7, 9 ou 10, **caractérisé en ce qu'**au moins une valeur constante est une valeur préalablement déterminée, de manière empirique, pour un capteur donné et pour un paramètre de caractérisation donné.

12. Procédé (100) l'une quelconque des revendications 1 à 7, **caractérisé en ce que** le paramètre de caractérisation est la densité de tissus osseux.

13. Utilisation du procédé selon l'une quelconque des revendications précédentes pour la caractérisation des tissus osseux d'une partie du corps d'un être vivant.

## Patentansprüche

1. Verfahren (100) zur Charakterisierung eines Knochengewebes mittels eines Bildgebungsgeräts (300), das einen Strahlensender (202) umfasst und einen Strahlenempfänger (204) einsetzt, wobei das Verfahren folgende Schritte umfasst:
- Erfassung (102) mindestens eines Bildes in Graustufen des Knochengewebes mittels des Bildgebungsgeräts zu einem sogenannten Messwert (MAS₁) für einen Sendeparameter bezüglich der gesendeten Strahlen,
- Messung (104) durch Analyse des Bildes:
• eines sogenannten Messwerts (GLA₁) für eine durchschnittliche Graustufe und
• eines sogenannten Messwerts für einen Charakterisierungsparameter (H₁) des Knochengewebes;
wobei das Verfahren **dadurch gekennzeichnet ist, dass** es außerdem folgende Schritte umfasst:
- Messung (104), durch Analyse des Bildes, eines sogenannten Messwerts (SD₁) für eine Normabweichung der Graustufen,
- die sogenannte Zielkalibrierung des Messwerts (H₁) für den Charakterisierungsparameter im Vergleich zu einem sogenannten, für den Sender vorbestimmten Zielwert (GLA_{cible}) für eine durchschnittliche Graustufe, wobei die Kalibrierung einen sogenannten Zielwert (H_{cible}) für den Charakterisierungsparameter zur Verfügung stellt,
- die sogenannte Festkalibrierung des Messwerts (SD₁) für die Normabweichung der Graustufen im Vergleich zu einem sogenannten, vorbestimmten Festwert (MAS_{fixe}) für den Sendeparameter, wobei die Kalibrierung einen sogenannten Festwert (SD_{fixe}) für die Normabweichung der Graustufen zur Verfügung stellt, und
- Bestimmung eines sogenannten Korrekturwerts (H_{corrigé}) für den Wert des Charakterisierungsparameters in Abhängigkeit von dem Zielwert (H_{cible}) für den Charakterisierungsparameter, und des Festwerts (SD_{fixe}) und eines Zielwerts (SD_{cible}) für die Normabweichung der Graustufen.

2. Verfahren (100) nach Anspruch 1, **dadurch gekennzeichnet, dass** der Schritt der Zielkalibrierung außerdem eine Bestimmung des Zielwerts für die Normabweichung der Graustufen in Abhängigkeit von dem Messwert (MAS₁) für den Sendeparameter und von einem vorbestimmten Zielwert (MAS_{cible}) für den Sendeparameter umfasst.

3. Verfahren (100) nach Anspruch 2, **dadurch gekennzeichnet, dass** der Schritt der Zielkalibrierung außerdem eine Bestimmung des Zielwerts (MAS_{cible}) für den Sendeparameter in Abhängigkeit von dem Messwert für den Sendeparameter (MAS₁), dem Messwert (GLA₁) für die durchschnittliche Graustufe und dem vorbestimmten Zielwert (GLA_{cible}) für die durchschnittliche Graustufe für den verwendeten Empfänger umfasst.

4. Verfahren (100) nach einem der vorhergehenden Ansprüche, **dadurch gekennzeichnet, dass** das Bild um einen sogenannten Interessenbereich herum erfasst wird, der vorab auf dem Bild durch mindestens einen anatomischen Marker erkannt wird.

5. Verfahren (100) nach einem der vorhergehenden Ansprüche, **dadurch gekennzeichnet, dass** der Festwert (SD_{fixe}) für die Normabweichung der Graustufen nach folgender Formel bestimmt wird: $S {D}_{fixe} = S {D}_{1} \left(\frac{MA {S}_{fixe}}{MA {S}_{1}}\right)$ wo:
- SD_{fixe} der Festwert für die Normabweichung der Graustufen ist,
- SD₁ der Messwert für die Normabweichung der Graustufen ist,
- MAS_{fixe} der Festwert für den Sendeparameter ist, und
- MAS₁ der Messwert für den Sendeparameter ist.

6. Verfahren (100) nach einem der Ansprüche 2 bis 5, **dadurch gekennzeichnet, dass** der Zielwert (SD_{cible}) für die Normabweichung der Graustufen nach folgender Formel bestimmt wird: $S {D}_{cible} = S {D}_{1} \left(\frac{MA {S}_{cible}}{MA {S}_{1}}\right)$ wo:
- SD_{cible} der Zielwert für die Normabweichung der Graustufen ist,
- SD₁ der Messwert für die Normabweichung der Graustufen ist,
- MAS_{cible} der Zielwert für den Sendeparameter ist, und
- MAS₁ der Messwert für den Sendeparameter ist.

7. Verfahren (100) nach einem der Ansprüche 3 bis 6, **dadurch gekennzeichnet, dass** der Zielwert (MAS_{cible}) für den Sendeparameter nach folgender Formel bestimmt wird: $MA {S}_{cible} = MAS \left(\frac{GL {A}_{cible} - cte}{GL {A}_{1} - cte}\right)$ wo:
- MAS_{cible} der Zielwert für den Sendeparameter ist,
- MAS₁ der Messwert für den Sendeparameter ist,
- GLA_{cible} der Zielwert für die durchschnittliche Graustufe ist,
- GLA₁ der Messwert für die durchschnittliche Graustufe ist, und
- cte eine Konstante ist.

8. Verfahren (100) nach einem der vorhergehenden Ansprüche, **dadurch gekennzeichnet, dass** der Charakterisierungsparameter der Parameter H ist, auch Hurst-Parameter genannt.

9. Verfahren (100) nach dem vorhergehenden Anspruch, **dadurch gekennzeichnet, dass** der Zielwert (H_{cible}) für den Charakterisierungsparameter nach folgender Formel bestimmt wird: ${H}_{cible} = {H}_{1} + ( \left(GL {A}_{cible} - GL {A}_{1}\right) . \left(\left(a {.10}^{- b}\right) - \left(c {.10}^{- d} . \left(GL {A}_{cible} + GL {A}_{1}\right)\right)\right)$ wo:
- H_{cible} der Zielwert für den Parameter H ist,
- H₁ der Messwert für den Parameter H ist,
- GLA_{cible} der Zielwert für die durchschnittliche Graustufe ist,
- GLA₁ der Messwert für die durchschnittliche Graustufe ist, und
- a, b, c und d Konstanten sind.

10. Verfahren (100) nach einem der Ansprüche 8 oder 9, **dadurch gekennzeichnet, dass** der Korrekturwert (H_{corrigé}) für den Charakterisierungsparameter nach folgender Formel bestimmt wird: ${H}_{corrigée} = {H}_{cible} + e \left(\frac{1}{S {D}_{fixe}} - \frac{1}{S {D}_{cible}}\right)$ wo:
- H_{corrigé} der Korrekturwert für den Parameter H ist,
- H_{cible} der Zielwert für den Parameter H ist,
- SD_{cible} der Zielwert für die Normabweichung der Graustufen ist,
- SD_{fixe} der Festwert für die Normabweichung der Graustufen ist, und
- e ein konstanter Wert ist.

11. Verfahren (100) nach einem der Ansprüche 7, 9 oder 10, **dadurch gekennzeichnet, dass** mindestens ein konstanter Wert ein vorab empirisch bestimmter Wert für einen bestimmten Empfänger und für einen bestimmten Charakterisierungsparameter ist.

12. Verfahren (100) nach einem der Ansprüche 1 bis 7, **dadurch gekennzeichnet, dass** der Charakterisierungsparameter die Dichte der Knochengewebe ist.

13. Anwendung des Verfahrens nach einem der vorhergehenden Ansprüche für die Charakterisierung der Knochengewebe eines Körperteils eines Lebewesens.

## Claims

1. Method (100) for the characterization of a bone tissue with an imaging device (300) comprising a radiation emitter (202) and utilizing a radiation sensor (204), said method being **characterized in that** it comprises the following steps:
- acquisition (102) of at least one grey scale image of said bone tissue with said imaging device at a value (MAS₁), known as measurement value, of an emission parameter relating to the emitted radiation,
- measurement (104), by analysis of said image:
• of a value (GLA₁) known as measured value, of a mean grey level, and
• of a value, known as measured value, of a characterization parameter (H₁) of said bone tissue;
said method being **characterized in that** it further comprises the following steps:
- measurement (104), by analysis of said image, of a value (SD₁), known as measured value, of a standard deviation of the grey levels,
- calibration, known as target calibration, of said measured value (H₁) of the characterization parameter with respect to a value (GLA_{cible}), known as target value, of a predetermined mean grey level for said sensor, said calibration producing a value (H_{cible}), known as target value, of said characterization parameter,
- calibration, known as fixed calibration, of said measured value (SD₁) of the standard deviation of the grey levels with respect to a value (MAS_{fixe}), known as fixed value, of the predetermined emission parameter, said calibration producing a value, known as fixed value (SD_{fixe}), of the standard deviation of the grey levels, and
- determination of a value (H_{corrigé}), known as corrected value, of the value of said characterization parameter, as a function of said target value (H_{cible}) of said characterization parameter, of said fixed value (SD_{fixe}), and of a target value (SD_{cible}) of the standard deviation of the grey levels.

2. Method (100) according to claim 1, **characterized in that** the target calibration step also comprises a determination of the target value of the standard deviation of the grey levels as a function of the measurement value (MAS₁) of the emission parameter and of a predetermined target value (MAS_{cible}) of said emission parameter.

3. Method (100) according to claim 2, **characterized in that** the target calibration step also comprises a determination of the target value (MAS_{cible}) of the emission parameter as a function of the measurement value of the emission parameter (MAS₁), of the measured value (GLA₁) of the mean grey level, and of the predetermined mean grey level target value (GLA_{cible}) for the sensor used.

4. Method (100) according to any one of the preceding claims, **characterized in that** the image is acquired around a region, known as the region of interest, previously identified on the image, by at least one anatomical marker.

5. Method (100) according to any one of the preceding claims, **characterized in that** the fixed value (SD_{fixe}) of the standard deviation of the grey levels is determined according to the following relationship: $S {D}_{fixe} = S {D}_{1} \left(\frac{MA {S}_{fixe}}{MA {S}_{1}}\right)$ with:
- SD_{fixe} the fixed value of the standard deviation of the grey levels,
- SD₁ the measured value of the standard deviation of the grey levels,
- MAS_{fixe} the fixed value of the emission parameter, and
- MAS₁ the measurement value of the emission parameter.

6. Method (100) according to any one of claims 2 to 5, **characterized in that** the target value (SD_{cible}) of the standard deviation of the grey levels is determined according to the following relationship: $S {D}_{cible} = S {D}_{1} \left(\frac{MA {S}_{cible}}{MA {S}_{1}}\right)$ with:
- SD_{cible} the target value of the standard deviation of the grey levels,
- SD₁ the measured value of the standard deviation of the grey levels,
- MAS_{cible} the target value of the emission parameter, and
- MAS₁ the measurement value of the emission parameter.

7. Method (100) according to any one of claims 3 to 6, **characterized in that** the target value (MAS_{cible}) of the emission parameter is determined according to the following relationship: $MA {S}_{cible} = MA {S}_{1} \left(\frac{GL {A}_{cible} - cte}{GL {A}_{1} - cte}\right)$ with:
- MAS_{cible} the target value of the emission parameter,
- MAS₁ the measured value of the emission parameter,
- GLA_{cible} the mean grey level target value,
- GLA₁ the mean grey level measured value, and
- cte a constant.

8. Method (100) according to any one of the preceding claims, **characterized in that** the characterization parameter is the parameter H, also known as the Hurst parameter.

9. Method (100) according to the preceding claim, **characterized in that** the target value (H_{cible}) of the characterization parameter is determined according to the following relationship: ${H}_{cible} = {H}_{1} + ( \left(GL {A}_{cible} - GL {A}_{1}\right) . \left(\left(a {.10}^{- b}\right) - \left(c {.10}^{- d} . \left(GL {A}_{cible} + GL {A}_{1}\right)\right)\right)$ with:
- H_{cible} the target value of the parameter H,
- H₁ the measured value of the parameter H,
- GLA_{cible} the mean grey level target value,
- GLA₁ the mean grey level measured value, and
- a, b, c and d, constant values.

10. Method (100) according to any one of claims 8 or 9, **characterized in that** the corrected value (H_{corrigé}) of the characterization parameter is determined according to the following relationship: ${H}_{corrigé} = {H}_{cible} + e \left(\frac{1}{S {D}_{fixe}} - \frac{1}{S {D}_{cible}}\right)$ with:
- H_{corrigé} the corrected value of the parameter H,
- H_{cible} the target value of the parameter H,
- SD_{cible} the target value of the grey level standard deviation,
- SD_{fixe} the fixed value of the standard deviation of the grey levels, and
- e a constant value.

11. Method (100) according to any one of claims 7, 9 or 10, **characterized in that** at least one constant value is a value previously determined empirically for a given sensor and for a given characterization parameter.

12. Method (100) according to any one of claims 1 to 7, **characterized in that** the characterization parameter is the density of bone tissues.

13. Use of the method according to any one of the preceding claims for the characterization of bone tissues of a part of the body of a living organism.
